# EUROPEAN PATENT SPECIFICATION

(11) **EP 0 924 061 B1**
(45) Date of publication and mention of the grant of the patent: **06.11.2002**
(21) Application number: 99103415.8
(22) Date of filing: 23.01.1992
(51) Int. Cl.: B32B 9/04, B32B 25/04, A61L 31/00, C08C 1/15, B29C 41/14

(54) **Latex article**
Latexformkörper
Article en latex

(43) Date of publication of application: 23.06.1999
(62) Divisional of application: 92300575.5
(73) Proprietor: Beck, Robin Thill, Los Angeles, California 90049 (US)
(72) Inventor: Beck, Robin Thill, Los Angeles, California 90049 (US); Solomons, Clive C., Denver, CO 80222 (US); Plunkett, Jerry D., Denver, Colorado 80210 (US); Smith, Clayton S., Golden, Colorado 80401 (US)
(74) Representative: Grünecker, Kinkeldey, Stockmair & Schwanhäusser Anwaltssozietät

(56) References cited:
- EP-A- 0 141 628
- EP-A- 0 300 814
- EP-A- 0 427 997
- WO-A-89/04647
- WO-A-90/01956
- WO-A-90/14048
- FR-A- 2 663 035
- US-A- 4 696 065
- US-A- 4 795 425

## Description

This invention relates to latex articles and their manufacture incorporating a biocidal material for preventing the transmission and dissemination of disease and is especially applicable to the manufacture of such articles as gloves and condoms.

Latex has long been used for the manufacture of gloves and condoms for the purpose of inhibiting the transmission of disease-producing microbes and other harmful agents. Both the chemical inertness and the physical density of cured latex make it difficult for molecules and microbes to pass through the structure of the latex material. Nevertheless, latex materials are known to possess imperfections in the form of pits, pores and holes which can facilitate the transmission of such microbes and harmful agents through the latex material.

WO-A-90/14048 discloses an article comprising a double latex layer and a protective substance or solution disposed there between.

It is the object of the present invention to provide an article by means of which it is possible to make a chemical barrier against the transmission of such microbes and other harmful agents through a membrane such as latex.

This object is achieved by a latex article having a biocide barrier, obtainable by a process comprising the steps of:
- applying onto a former first coating consisting essentially of of liquid latex and free of biocide;
- applying to the first coating of liquid latex a second coating consisting essentially of a biocide effective as a coagulant for liquid latex; and
- applying to said second coating a third coating consisting essentially of liquid latex and free of biocide.

It is preferred that said third coating is thicker than the first coating and also that the first coating is leached with water prior to application of the second coating. It is further preferred that the second coating is dried before applying the third coating, further that said first coating is applied by dipping the former into liquid latex, and even further that the second coating is applied onto the first coating when the latex in the first coating is in a wet gel state. Furthermore, it is preferred that the second coating is applied by spraying a biocide solution. Also preferred is that said second coating is applied by dipping the former into a biocide solution.

It is further preferred that the biocide is applied as a solution having a concentration of 0.10 to 5 percent by weight, even further preferred that said third coating is applied by dipping the former in a vat of liquid latex, and even further preferred that the biocide is selected from chlorhexidine, dextran sulfate, triclosan, benzalkonium, betadyne, gentian violet, acriflavine and acridine dyes, mercurochrome, silver salts, and an extract of blue-green algae.

Preferably, the weight ratio of the biocide solution to the first latex coating is from 0.05 to 3, and also preferably the weight ratio of the biocide solution in the second coating to the latex applied in the third coating is between 0.05 and 0.3. It is further preferred that the biocide is bonded to and is permanently diffused within the surface of the latex membrane, further preferred that the latex article is in the form of a glove, condom, diaphragm, slipper, overshoe, sterile band, catheter, tubing, drape, gut opening, mouthpiece, nipple, intra gastric nasal tube, kidney shunt, dam for teeth, brace for teeth, sub-clavian vein and artery shunt or colostomy bag, and further preferred that the liquid latex is selected among natural and synthetic latex, and more preferred that the synthetic latex is selected among solvent cast membranes, elastomers, polymers and synthetic latex known in the art as natural skin.

The present invention provides a method of forming a chemical barrier against the transmission of disease-causing microbes and other harmful agents through a membrane such as latex. In the method, a mold or former is coated with a biocide/coagulant which is dried, then the former is dipped into liquid latex. In an alternative procedure, the former is initially dipped into liquid latex which is allowed to gel before being dipped into the biocide/coagulant. The entire coating on the former is then cured. Alternatively, the biocide may be sprayed or otherwise applied onto the former or the gelled latex.

The present invention concerns the forming of a chemical barrier against disease-causing microbes and other harmful agents through a membrane fashioned from natural latex or from a synthetic latex material such as those known as natural skin, solvent cast membranes, elastomers, and polymers formed by curing the material from a liquid state. For convenience, the preferred aspects of the invention will be described with reference to a latex material. The latex material may be fashioned as a glove, condom, diaphragm, slipper, overshoe, sterile band, catheter, tubing, diaphragm, drape, gut opening, mouthpiece, baby nipple, intragastric nasal tube, nasal gastric tube, kidney shunt, rubber dam for teeth, plastic brace for teeth, sub-clavian vein or artery shunt, colostomy bag or any other product. Normally such latex products will be adapted for use in juxtaposition to a person's or animal's skin.

As used in the context of the present application, the term "biocide" means a substance or material which renders the disease-producing characteristic or harm-causing characteristic of a microbe or harmful agent ineffective substantially upon contact or shortly after contact of the microbe or harmful agent with the biocide. Examples of suitable biocides are dextran sulphate, nonoxynol-9, benzalkonium, betadyne, gentian violet, acriflavine and acridine dyes, mercurochrome, silver salts and an extract of blue green algae. Other examples of suitable biocides are set out in the list below.

Latex products, such as latex gloves and condoms, are conventionally formed by either a single dip or a double dip process. In these processes, a mold or former in the shape of the desired latex product is initially covered with a chemical coagulant, which allows a relatively thick, uniform, continuous layer of latex to be deposited on the former. The coagulant is dried on the former, and then the former is dipped into a vat of liquid latex for an appropriate period of time, whereby a film of latex is picked up on the surface of the former. The latex is allowed to wet gel into a sticky or tacky state and while in the gel state, is leached in a warm water rinse or bath. The warm water removes the coagulant as well as any residual ammonia or potassium hydroxide which might be present. In order to increase the thickness of latex, the process may be repeated so that a second film of latex is built up integrally over the first layer of latex. Usually the second layer is less thick than the first layer.

In accordance with the invention, the coagulant is selected so as to have biocidal properties so that the final latex product incorporates a biocide barrier. The method permits such latex products to be made by the general procedure described above and by the alternative procedure of initially applying a first liquid latex coating to the mold or former and then applying the biocide/coagulant to that first liquid latex coating before applying one or more further liquid latex coatings over the biocide/coagulant.

A biocide barrier can be incorporated into a latex product in the above manner, as follows. After the first dip of the former into the liquid latex and after the preferred warm water leach in a conventional manner, the former with the liquid latex in a wet gel state is dipped into a 0.10 to 5% by weight solution of gentian violet, for example a 0.33% by weight solution in water. The biocide solution coats the liquid latex. Alternatively, the biocide solution may be sprayed or otherwise applied to the wet gel latex. The biocide solution coating is then dried. Thereafter, the former is dipped again into a vat of liquid latex, may be leached again, and then the entire coating on the former is cured by drying.

It appears that the biocide solution acts as a coagulant, and causes the second layer of latex to be relatively thicker than is normally achieved without any biocide solution.

The amount of biocide solution applied either to the former or to the initial latex coating on the former is preferably such that the weight ratio of that biocide solution coating to the subsequent latex coating is 0.05 to 0.3.

A solution of 1.5% by weight of for example, gentian violet in water has also been used in this process, with slightly less desirable uniformity in thickness of the coatings. Nevertheless, it is believed that concentrations of up to about 5% by weight of gentian violet in water or in water and alcohol with or without other cosolvents can be advantageously used in the process.

It has been observed that the maximum amount of biocide solution picked-up by the wet gel latex coating the former is about 0.2 grams of solution per gram of latex.

It is believed that the biocide is bonded to and is permanently diffused within the surface of the latex, thereby substantially filling the pores and other imperfections of the latex.

The following biocides are believed to be equally suitable to those described above for use in the invention:-
halogen and halogen compounds - chlorine and iodine;
phenolic compounds;
alcohols;
oxidants;
chlorhexidine;
nitrogen compounds;
surface active agents, such as
   quaternary ammonium compounds,
   acid anionic compounds,
   amphoteric compounds,
   heavy metals and
   dyes, e.g. crystal violet;
antibiotics;
HC BLUE NO. 2
   (N¹,N⁴,N⁴,-(2-hydroxyethyl)-2-nitro-p-phenylene-diamine);
NONOXYNOL-2
   (polyoxyethylene (2) nonyl phenyl ether);
NONOXYNOL-4 and -8;
D & C RED NO. 22
   (eosine YS);
D & C RED NO. 37
   (rhodamine B-stearate);
D & C RED NO. 37 CALCIUM LAKE
   (rhodamine B stearate solvent);
and the following products listed according to their recommended or generally accepted biocidal applications:-

### Antimicrobial Soaps:

Cloflucarban
Para-chloro-mein-xylenof
Povidone-iodine complex
1.5 percent phenol or less aqueous/alcoholic
Triclocarbon
Tricloean

### Health-care Peronnel Handwash:

Bensalkonium chloride
Benzathonium chloride
Clofluearban
Hexylesorinal
Iodine complexed with phophate eater of alkyaryloxy polyethlene glycol
Methyl-benzethonium chloride
Nonyl phenoxypoly (ethyleneoxy) ethanol-iodine
Para-chloro-meta-xylenol
Povidene-iodine complex
1.5 percent phenol or less aqueous/alcoholic
Poloxamer-iodine complex
Tricloearban¹
Undecoylium chloride-iodine complex

### Patient preoperative skin preparation

Benzalkonium chloride
Benzathonium chloride
Hexylresorcinol
Iodine complexed with phosphate ester of alkylaryloxy polyethylene glycol
Methylbenxethonium chloride
Nonyl phenoxypoly (ethyleneoxy) ethanoilodine
Para-thloro-meta-xylenol
1.5 percent phenol or less aqueous/alcoholic
Poloxamer-iodine complex
Povidene-iodine complex
Undecoylium chloride-iodine complex

### Skin antiseptic

Benzalkonium chloride
Benzathonium chloride
Hexylresorcinol
Iodine complexed with phosphate ester of alkylaryloxy polyethylene glycol
Iodine tincture
Methyl-bonzethonium chloride
Nonyl phenoxypoly (ethylencoxy) ethanoliodine
Para-chloro-meta-xylenol
1.5 percent phenol or less aqueous/alcoholic
Poloxamer-iodine complex
Povidene-iodine complex
Triclosan
Triple Dye
Undecoylium chloride-iodine complex

### Skin wound cleanser

Cloflutarban¹
Iodine complexed with phosphate ester of alkylaryloxy polyethylene glycol
Iodine tincture
Nonyl phenoxypoly (ethyleneoxy) ethanoliodine
Para-chloro-meta-xylenol
1.5 percent phenol or less aqueous/alcoholic
Poloxamer-iodine complex
Povidene-iodine complex
Tricloearban¹
Triclosan
Undecoylium chloride-iodine complex

### Skin wound protectant

Benzalkonium chloride
Benzathonium chloride
Hexylresorcinol
Iodine complexed with phosphate ester of alkylaryloxy polyethylene glycol
Iodine tincture
Methyl-bonzethonium chloride
Nonyl phenoxypoly (ethylencoxy) ethanoliodine
Para-chloro-meta-xylenol

## Claims

1. A latex article having a biocide barrier, obtainable by a process comprising the steps of:
- applying onto a former a first coating consisting essentially of liquid latex and free of biocide;
- applying to the first coating of liquid latex a second coating consisting essentially of a biocide effective as a coagulant for liquid latex; and
- applying to said second coating a third coating consisting essentially of liquid latex and free of biocide.

2. The latex article according to claim 1, wherein in said process the third coating is thicker than the first coating.

3. The latex article according to any of claims 1 or 2, wherein in said process the first coating is leached with water prior to application of the second coating.

4. The latex article according to any of claims 1 to 3, wherein in said process the second coating is dried before applying the third coating.

5. The latex article according to any of claims 1 to 4, wherein in said process said first coating is applied by dipping the former into liquid latex.

6. The latex article according to any of claims 1 to 5, wherein in said process the second coating is applied onto the first coating when the latex in the first coating is in a wet gel state.

7. The latex article according to any of claims 1 to 6, wherein in said process the second coating is applied by spraying a biocide solution.

8. The latex article according to any of claims 1 to 6, wherein in said process said second coating is applied by dipping the former into a biocide solution.

9. The latex article according to any of claims 1 to 8, wherein in said process the biocide is applied as a solution having a concentration of 0.10 to 5 percent by weight.

10. The latex article according to any of claims 1 to 9, wherein in said process said third coating is applied by dipping the former in a vat of liquid latex.

11. The latex article according to any of claims 1 to 10, wherein the biocide is selected from chlorhexidine, dextran sulfate, triclosan, benzalkonium, betadyne, gentian violet, acriflavine and acridine dyes, mercurochrome, silver salts, and an extract of blue-green algae.

12. The latex article according to any of claims 1 to 11, wherein in said process the weight ratio of the biocide solution to the first latex coating is from 0.05 to 3.

13. The latex article according to any of claims 1 to 12, wherein in said process the weight ratio of the biocide solution in the second coating to the latex applied in the third coating is between 0.05 and 0.3.

14. The latex article according to any of claims 1 to 13, wherein the biocide is bonded to and is permanently diffused within the surface of the latex membrane.

15. The latex article according to any of claims 1 to 14, which is in the form of a glove, condom, diaphragm, slipper, overshoe, sterile band, catheter, tubing, drape, gut opening, mouthpiece, nipple, intra gastric nasal tube, kidney shunt, dam for teeth, brace for teeth, sub-clavian vein and artery shunt or colostomy bag.

16. The latex article according to any of claims 1 to 15, wherein the liquid latex is selected among natural and synthetic latex.

17. The latex article according to claim 16, wherein the synthetic latex is selected among solvent cast membranes, elastomers, polymers and synthetic latex known in the art as natural skin.

## Patentansprüche

1. Latexartikel mit einer Biozidbarierre, erhältlich durch ein Verfahren, umfassend die folgenden Schritte:
- Aufbringen einer ersten Schicht, die im wesentlichen aus flüssigem Latex besteht und frei von Bioziden ist, auf eine Form;
- Aufbringen einer zweiten Schicht, die im wesentlichen aus einem Biozid besteht, das als Koagulant für flüssigen Latex wirksam ist, auf die erste Schicht aus flüssigem Latex; und
- Aufbringen einer dritten Schicht, die im wesentlichen aus flüssigem Latex besteht und frei von Bioziden ist, auf diese zweite Schicht.

2. Latexartikel nach Anspruch 1, worin in besagtem Verfahren die dritte Schicht dicker ist als die erste Schicht.

3. Latexartikel nach Anspruch 1 oder 2, worin in besagtem Verfahren die erste Schicht mit Wasser ausgelaugt wird, bevor die zweite Schicht aufgebracht wird.

4. Latexartikel nach einem der Ansprüche 1 bis 3, worin in besagtem Verfahren die zweite Schicht getrocknet wird, bevor die dritte Schicht aufgebracht wird.

5. Latexartikel nach einem der Ansprüche 1 bis 4, worin in besagtem Verfahren die erste Schicht durch Eintauchen der Form in flüssigen Latex aufgebracht wird.

6. Latexartikel nach einem der Ansprüche 1 bis 5, worin in besagtem Verfahren die zweite Schicht auf die erste Schicht aufgebracht wird, wenn der Latex der ersten Schicht den Zustand eines nassen Gels hat.

7. Latexartikel nach einem der Ansprüche 1 bis 6, worin in besagtem Verfahren die zweite Schicht durch Aufsprühen einer Biozidlösung aufgebracht wird.

8. Latexartikel nach einem der Ansprüche 1 bis 6, worin in besagtem Verfahren die zweite Schicht durch Eintauchen der Form in eine Biozidlösung aufgebracht wird.

9. Latexartikel nach einem der Ansprüche 1 bis 8, worin in besagtem Verfahren das Biozid als Lösung mit einer Konzentration von 0,10 bis 5 Gew.-% aufgebracht wird.

10. Latexartikel nach einem der Ansprüche 1 bis 9, worin die dritte Schicht in besagtem Verfahren durch Eintauchen der Form in einen Bottich mit flüssigem Latex aufgebracht wird.

11. Latexartikel nach einem der Ansprüche 1 bis 10, worin das Biozid gewählt wird aus Chlorhexidin, Dextransulfat, Triclosan, Benzalkonium, Betadyne, Gentianaviolett, Acriflavin und Acridin-Farbstoffen, Mercurochrom, Silbersalzen und einem Extrakt aus blaugrünen Algen.

12. Latexartikel nach einem der Ansprüche 1 bis 11, worin in besagtem Verfahren das Gewichtsverhältnis der Biozidlösung zu der ersten Latexschicht 0,05 bis 0,3 beträgt.

13. Latexartikel nach einem der Ansprüche 1 bis 12, worin in besagtem Verfahren das Gewichtsverhältnis der Biozidlösung in der zweiten Schicht zu dem in der dritten Schicht aufgebrachten Latex zwischen 0,05 und 0,3 liegt.

14. Latexartikel nach einem der Ansprüche 1 bis 13, worin in besagtem Verfahren das Biozid an die Oberfläche der Latexmembran gebunden ist und durch Diffusion in der Oberfläche der Latexmembran permanent fein verteilt ist.

15. Latexartikel nach einem der Ansprüche 1 bis 14, in der Form eines Handschuhs, eines Kondoms, eines Diaphragmas, eines Slippers, eines Überschuhs, eines sterilen Bands, eines Katheters, eines Schlauchs, einer Umhüllung, einer Darmöffnung (gut opening), eines Mundstücks, eines Nippels, eines intragastrischen Nasalschlauchs, eines Lebershunts, eines Kofferdams für Zähne, einer Zahnspange, eines subklavikularen Venen- oder Arterienshunts oder eines Kolostomie-Beutels.

16. Latexartikel nach einem der Ansprüche 1 bis 15, wobei der flüssige Latex ausgewählt ist unter natürlichem und synthetischem Latex.

17. Latexartikel nach Anspruch 16, wobei der synthetische Latex ausgewählt ist unter Lösongsmittelgießmembranen, Elastomeren, Polymeren und synthetischem Latex bekannt als natürliche Haut.

## Revendications

1. Article en latex ayant une barrière biocide, susceptible d'être obtenu par un procédé comprenant les étapes consistant :
- à appliquer sur une forme un premier revêtement essentiellement constitué par un latex liquide et exempt de biocide ;
- à appliquer sur le premier revêtement de latex liquide un deuxième revêtement essentiellement constitué par un biocide efficace en tant que coagulant pour le latex liquide ; et
- à appliquer sur ledit deuxième revêtement un troisième revêtement essentiellement constitué par un latex liquide et exempt de biocide.

2. Article en latex selon la revendication 1, dans lequel, dans ledit procédé, le troisième revêtement est plus épais que le premier revêtement.

3. Article en latex selon l'une quelconque des revendications 1 ou 2, dans lequel, dans ledit procédé, le premier revêtement est lessivé avec de l'eau avant l'application du deuxième revêtement.

4. Article en latex selon l'une quelconque des revendications 1 à 3, dans lequel, dans ledit procédé, le deuxième revêtement est séché avant d'appliquer le troisième revêtement.

5. Article en latex selon l'une quelconque des revendications 1 à 4, dans lequel, dans ledit procédé, ledit premier revêtement est appliqué en immergeant la forme dans un latex liquide.

6. Article en latex selon l'une quelconque des revendications 1 à 5, dans lequel, dans ledit procédé, le deuxième revêtement est appliqué sur le premier revêtement lorsque le latex dans le premier revêtement est à l'état de gel humide.

7. Article en latex selon l'une quelconque des revendications 1 à 6, dans lequel, dans ledit procédé, le deuxième revêtement est appliqué en pulvérisant une solution de biocide.

8. Article en latex selon l'une quelconque des revendications 1 à 6, dans lequel, dans ledit procédé, ledit deuxième revêtement est appliqué en immergeant la forme dans une solution de biocide.

9. Article en latex selon l'une quelconque des revendications 1 à 8, dans lequel, dans ledit procédé, le biocide est appliqué sous la forme d'une solution ayant une concentration de 0,10 à 5 % en poids.

10. Article en latex selon l'une quelconque des revendications 1 à 9, dans lequel, dans ledit procédé, ledit troisième revêtement est appliqué en immergeant la forme dans une cuve de latex liquide.

11. Article en latex selon l'une quelconque des revendications 1 à 10, dans lequel le biocide est choisi parmi la chlorhexidine, le sulfate de dextrane, le triclosan, le benzalkonium, la betadyne, le violet de gentiane, l'acriflavin et les colorants acridines, le mercurochrome, les sels d'argent et un extrait d'algue bleue.

12. Article en latex selon l'une quelconque des revendications 1 à 11, dans lequel, dans ledit procédé, le rapport pondéral de la solution de biocide au premier revêtement de latex est de 0,05 à 3.

13. Article en latex selon l'une quelconque des revendications 1 à 12, dans lequel, dans ledit procédé, le rapport pondéral de la solution de biocide dans le deuxième revêtement au latex appliqué dans le troisième revêtement est entre 0,05 et 0,3.

14. Article en latex selon l'une quelconque des revendications 1 à 13, dans lequel le biocide est lié à la surface de la membrane en latex et est diffusé en permanence dans celle-ci.

15. Article en latex selon l'une quelconque des revendications 1 à 14, qui est sous la forme d'un gant, d'un préservatif, d'un diaphragme, d'un chausson, d'un couvre-chaussure, d'une bande stérile, d'un cathéter, d'un tube, d'un drap, d'une ouverture intestinale, d'un protège-dents, d'une tétine, d'une sonde gastrique, d'un pontage rénal, d'une digue dentaire, d'une bague dentaire, d'une veine sous-clavière et d'un pontage artériel ou d'une poche pour colostomie.

16. Article en latex selon l'une quelconque des revendications 1 à 15, dans lequel le latex liquide est choisi parmi un latex naturel et un latex synthétique.

17. Article en latex selon la revendication 16, dans lequel le latex synthétique est choisi parmi les membranes coulées avec un solvant, les élastomères, les polymères et un latex synthétique connu dans la technique sous le nom de peau naturelle.
